# EUROPEAN PATENT SPECIFICATION

(11) **EP 1 042 017 B1**
(45) Date of publication and mention of the grant of the patent: **20.11.2002**
(21) Application number: 98963006.6
(22) Date of filing: 09.12.1998
(51) Int. Cl.: A61L 15/46, A61F 13/15

(54) **COMPACTED ODOUR CONTROL PARTICLES FOR USE IN ABSORBENT ARTICLES**
VERDICHTETE GERUCHSKONTROLLTEILCHEN ZUM GEBRAUCH IN ABSORBIERENDEN ARTIKELN
PARTICULES DESODORISANTES COMPACTEES POUR ARTICLES ABSORBANTS

(30) Priority: 18.12.1997 EP 97122343
(43) Date of publication of application: 11.10.2000
(73) Proprietor: THE PROCTER & GAMBLE COMPANY, Cincinnati, Ohio 45202 (US)
(72) Inventor: CARLUCCI, Giovanni, I-66100 Chieti (IT); GAGLIARDI, Ivano, I-65123 Pescara (IT); DIVO, Michael, D-61381 Friedrichsdorf (DE)
(74) Representative: Kremer, Véronique
(86) International application number: US9826170
(87) International publication number: WO99030754

(56) References cited:
- WO-A-91/12031
- WO-A-94/28107

## Description

### Background of the Invention

The present invention relates to odour controlling particles which find application in absorbent articles, in particular sanitary napkins and panty liners. Odour control particles are provided to combat a broad range of odourous compounds and are provided in a particulate form by compaction without the necessity of a binder. The present invention hence provides a simplified method of manufacture for odour control particles and thereby also provide odour control particles delivering an improved odour control performance benefit.

### Field of the Invention

Whilst the primary focus of absorbent articles remains the ability of these articles to absorb and retain fluids, another important area of development in this field is the control of odourous compounds contained within the absorbed fluids or their degradation products. There are a wide range of compounds which may be present in an absorbent article during use which result in the formation of malodourous. These compounds include fatty acids, ammonia, amines, sulphur containing compounds and ketones and aldehydes.

The art is replete with descriptions of various odour controlling agents for use in absorbent articles in order to address the problem of malodour formation. These agents can typically be classified according to the type of odour the agent is intended to combat. Odours may be classified as being acidic, basic or neutral. Acidic odour controlling agents have a pH greater than 7 and typically include inorganic carbonates, bicarbonates, phosphates and sulphates. Basic odour controlling agents have a pH of less than 7 and include compounds such as citric acid, boric acid and maleic acid.

The most commonly utilised odour controlling agents are the neutral odour control agents which have a pH of approximately 7. Examples of these known types of compounds include activated carbons, clays, zeolites, silicas, starches and certain combinations thereof. Examples of such agents are described in EPO 348 978 which discloses an absorbent article comprising an odour control system wherein the neutral odour controlling particles are selected from carbon, clays, silicas, zeolites and molecular sieves. Also in EPO 510 619 which relates to an absorbent article comprising odour control complex including a combination of at least 2 agents selected from a group including zeolites and silica gels. Similarly, WO 91/11977 and WO 91/12030 disclose certain combinations of zeolites and absorbent gelling materials.

However, many of these neutral odour control agents and combinations thereof have certain associated disadvantages therewith. For example silica and silica molecular sieves are considered expensive odour control agent components. On the other hand activated carbon which has been noted in the art as providing particularly effective odour control, is not favoured due to its black appearance, which is considered unacceptable by consumers. Zeolites which unlike carbon do not have a negative aesthetic profile, are not considered to deliver effective odour control over a broad range of odour types. Moreover, another disadvantage is that the more effective types of odour control zeolites, the so-called intermediate and high ratio SiO₂/AlO₂ zeolites are also particularly expensive.

Another particular problem especially acute for multi-component odour control systems, particularly those comprising zeolite is that it is essential in such systems that the components are sufficiently blended prior to their incorporation into the absorbent article. This is in order that the odour control system can be satisfactorily homogeneously incorporated within the absorbent article at the desired location and thereby deliver the maximum odour control benefit.

Furthermore, yet another problem related to the satisfactory incorporation of many of the odour control materials, particularly zeolites, within absorbent articles, is concerned with the particles size of the odour control materials. In order to be readily and homogeneously incorporated into the absorbent articles without the formation of dust the odour control particles are required to have certain dimensions. The formation of dust is considered as a safety hazard and also results in the reduction of the quality of the laminate structure in which the odour control particles are typically incorporated. This typically occurs by the odour control particles deactivating the adhesive and hindering bonding of the laminate and also thereby allowing the particles to escape from the laminate and the article. Hence it is desirable that the odour control materials should preferably be provided such that they have a minimum particle size. However, in order to achieve this the use of specific manufacturing or processing techniques are often required.

In order to address the problem of dust formation, it has been suggested for example to adhere the particulate odour controlling agents to the surface of larger particles of absorbent gelling materials utilizing moisture. The resulting particles are less dusty, easier to handle and exhibit improved odour control. However, it has also been observed that such absorbent gelling material particles can only be loaded with up to about 10% by weight of odour control agent using water.

Attempts to increase the amount of odour controlling agents in such particles comprising absorbent gelling materials as for example described in WO 91/12029 have resided in the utilization of a binder material within the particle. Typically, this can be achieved by using a fluidized bed apparatus known as agglomeration. However, a problem related to using binder materials within odour control particles is that the binders may at least partially physically inactivate the odour control active materials. This may occur by physical covering or blocking of the pore sites of the odour control active materials, thereby rendering the sites inaccessible to the odourous compounds. Alternatively, the binder materials may chemically inactive the odour control active materials. Moreover, whilst the amount of odour control active material that can be incorporated within the particle may be increased compared to the process of using moisture as a binder, the particles typically require a binder content of about 10% by weight, in order to form the particles. Obviously, the are occupied by the binder cannot be occupied by odour control active materials.

Furthermore, whilst it is highly desirable from a manufacturing point of view to incorporate agglomerated odour control particles within absorbent articles these particles are thus bonded to such a degree that the particles will retain their integrity during the entire use of the article, even after contact with the liquid discharge. This is however highly undesirable as consequently a large proportion of the odour control active material will not contact the liquid discharge and the entire odour control capacity of the components will not be realized.

Hence there exists a need to provide odour control particles which do not necessitate the presence of a binder material and which retain their structural integrity such that they can be easily and readily handled and positioned in the desired location of the absorbent articles. In addition there also exists a need to provide odour control particles which will preferably separate into the individual odour control active material components during the use of the absorbent article.

It has now been unexpectedly found that these objectives can be met by providing odour control particles comprising odour control active materials, the particles having been formed by dry pressure compaction which does not require the presence of a binder.

### Summary of the Invention

The present invention hence relates to odour control particles which find application in absorbent articles such as sanitary napkins, diapers, incontinence products and perspiration pads. The odour control particles of the present invention comprise at least one odour control active material which have a density less than that of the density of the odour control particles and, which are dry pressure compacted to form the odour control particles. The compacted odour control particles of the present invention offer the advantage that unlike agglomerated particles, the particles can be formed in the absence of a binder and readily separate into the individual odour control active material components upon use in an absorbent article. This allows particles to be provided which contain only odour control active materials. The present invention furthermore also relates to the process of formation of the odour control particles by dry pressure compacting.

### Detailed Description of the Invention

The present is thus related to the provision of odour control particles for use in absorbent articles which comprises odour control actives which are dry pressure compacted to form the odour control particles.

According to the present invention the odour control particles comprise any odour control active materials known in the art for that purpose. Suitable odour control active materials for use herein include zeolites, silica, carbon, chelants such as for example ethylene diaminetetraacetic acid, cyclodextrin, absorbent gelling materials (AGM), antimicrobial agents, perfuming ingredients, masking agents, and mixtures thereof. Particularly preferred are zeolites, silica, AGM, chelants and mixtures thereof. Most preferred are mixtures of zeolite, silica and AGM. Suitable zeolites for use herein may be naturally derived or synthetically manufactured, although the synthetic zeolites are preferred. Suitable zeolites for use herein thus include zeolite A, zeolite P, zeolite Y, zeolite X, zeolite DAY, zeolite ZSM-5, or mixtures thereof Most preferred are zeolite A, zeolite Y or mixtures thereof. Preferred cations may be selected from group I and II metals and may also include heavy metals such as for example zinc, silver and copper. The zeolite is also preferably hydrophobic and this can be typically achieved by increasing the molar ratio of the SiO₂ to AlO₂ content such that the ratio of x to y is at least 1, preferably from 1 to 500, most preferably from 1 to 6. Similarly, synthetic and naturally derived silica i.e silicon dioxide may also be utilised. Most preferred is silica gel. However any other known odour control active and combination thereof can be selected by the skilled man depending on the odours which the odour control active materials should combat.

According to the present invention the odour control particles may be prepared using a simple but effective method which involves high pressure compacting, using press rolls of the odour control active materials, as described more fully in the examples below. This method provides an improved alternative method to conventional wet agglomeration techniques which require a binder material.

According to the present invention the odour control particles are prepared in the absence of a binder. The term binder as used herein refers to binder materials well known in commerce under various trade names such as GELFORM, PURAGEL, LAVERAL, MALTRIN and METHOCEL. In general these binders are soluble or dispersible in water or body fluids such as blood and urine. Chemically such binders comprise various starch, cellulose, modified starch, modified cellulose, gum acacia, gum arabic, soluble gelatine materials or mixtures thereof

According to the present invention the compacted odour control particles may have any particle size as desired and it can thus be selected according to the end use of the particles envisioned. Typically for absorbent article applications such as sanitary napkins, the odour control particles should preferably have a mean particle size such that no more than 5 % of the particles are greater than 1.7 mm in diameter and not more than 5 % are less than 0.15 mm in diameter. Preferably, the mean particle size is from 0.1 mm to 2.0 mm, preferably from 0.2 mm to 0.7 mm, most preferably from 0.3 mm to 0.5 mm. The particle size can be readily determined by sieve analysis.

The odour control particles of the present invention find particular utility within absorbent articles. Particularly suitable absorbent articles include sanitary napkins, panty liners, diapers, incontinence products and perspiration pads. In particular the present invention finds application in sanitary napkins and panty liners.

### Absorbent article

In order to more fully assess the utility of the process of the present invention a description of a typical disposable absorbent article follows. Typically absorbent articles comprise a liquid permeable topsheet, a liquid impervious backsheet and an absorbent structure positioned in-between the topsheet and backsheet.

### The topsheet

The topsheet is compliant, soft feeling, and non-irritating to the wearer's skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness. A suitable topsheet can be manufactured from a wide range of materials such as woven and non woven materials; polymeric materials such as apertured formed thermoplastic films, apertured plastic films, and hydroformed thermoplastic films; and thermoplastic scrims. Suitable woven and non woven materials can be comprised of natural fibers (e.g., wood or cotton fibers), synthetic fibers (e.g., polymeric fibers such as polyester, polypropylene, or polyethylene fibers) or from a combination of natural and synthetic fibers or bi-/multi-component fibers.

Preferred topsheets for use in the present invention are selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,394. Particularly preferred micro apertured formed film topsheets are disclosed in U.S. patent 4,609,518 and U.S. patent 4,629,643. A preferred topsheet for the present invention comprises the formed film described in one or more of the above patents and marketed on sanitary napkins by The Procter & Gamble Company of Cincinnati, Ohio as "DRI-WEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The body surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT-publication WO 93/09741. Alternatively, the body surface of the topsheet can be made hydrophilic by treating it with a surfactant such as is described in U.S. 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

The topsheet typically extends across the whole of the absorbent structure and outside the area coextensive with the absorbent structure. The topsheet can extend and form part or all of the preferred side flaps, side wrapping elements or wings.

When referring to the topsheet a multi layer structure or a mono layer structure is contemplated. The hybrid topsheet mentioned above is such a multi layer design but other multi layer topsheets such as primary and secondary topsheet designs are also considered.

### Absorbent core

According to the present invention the absorbent cores suitable for use in herein may be selected from any of the absorbent cores or core system known in the art. As used herein the term absorbent core refers to any material or multiple material layers whose primary function is to absorb, store and distribute fluid.

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components.

### a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers-the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials in addition to the absorbent gelling material that may be provided within the odour control particles of the present invention, usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water. Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

### Backsheet

The backsheet primarily prevents the exudates absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance.

According to a preferred embodiment of the present invention the backsheet of the absorbent article is moisture vapour permeable and thus comprises at least one gas permeable layer. Suitable gas permeable layers include 2 dimensional, planar micro and macro-porous films, macroscopically expanded films, formed apertured films and monolithic films. According to the present invention the apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable 2 dimensional planar layers of the backsheet may be made of any material known in the art, but are preferably manufactured from commonly available polymeric materials. Suitable materials are for example Gortex (TM) or Sympatex (TM) type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein the term 2 dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3, 929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core.

Suitable macroscopically expanded films for use herein include films as described in for example in US 4 637 819 and US 4 591 523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours international S.A, Switzerland, such as Pebax™, available from Elf Atochem (France) and Estane™ available from BF Goodrich (Belgium).

Particularly preferred backsheets for the present invention comprise at least two layers comprising at least one layer selected from the above, such as microporous and apertured formed films and an additional layer which may also be selected from the above listed backsheets or may be a fibrous woven or nonwoven. The most preferred breathable backsheet construction comprises a microporous film and an apertured formed film or a microporous film and a hydrophobic nonwoven or woven textile.

Thus, in addition to the components described herein above, the absorbent article may also comprise all those features and parts which are typical for products in the context of their intended use such as wings and side flaps, undergarment adhesive means, release paper, wrapping elements, fastening means and the like.

According to the present invention the absorbent article is constructed by joining the various elements such as topsheet, backsheet and absorbent core by any means well known in the art. For example the backsheet and/ or topsheet may be joined to the absorbent core or to each other by a uniform continuous layer of adhesive, a patterned layer of adhesive, or an array of separate lines, spirals or spots of adhesive. Alternatively, the elements may be joined by heat bonds, pressure bonds, ultra sonic bonds, dynamic mechanical bonds or any other suitable joining means known in the art and any combination thereof.

The odour control particles may be incorporated into the absorbent article by any of the methods disclosed in the art. Typically the odour control particles are positioned within the absorbent core structure, for example the particles may be layered on the core of the absorbent article or mixed within the fibres of the absorbent core. The odour control system is preferably incorporated between two layers of air laid cellulose tissue. Optionally the particles may be bonded between two cellulose tissue layers with, for example, a hot melt adhesive or any suitable bonding system. The adhesive is preferably also placed on the edges of the laminate to ensure that the edges of the laminate adhere to one another so that any loose odour control particles not attached to the laminate cannot fall out of the laminate. More preferably the odour control particles is incorporated in a layered structure in accordance with the disclosure of WO 94/01069.

According to the present invention the amount of the odour control particles which may be used in the absorbent article can be readily determined by the skilled person bearing in mind the type of absorbent articles and the dimensions of the absorbent article. For example, the absorbent article may comprise from 0.2g to 15g, preferably from 0.5g to 5g, most preferably from 1.5g to 2.5g of said odour control particles. Typically sanitary napkins will comprise from 0.5g to 10g, preferably from 1g to 8g, most preferably from 1.5g to 6.5g of said odour control particles.

### Preparation of the odour control particles

According to the present invention the odour control particles may be prepared using a simple but effective method. The method involves high pressure compaction of odour control active material, using press rolls, as described more fully in the examples below. This method provides an improved alternative to conventional wet agglomeration techniques which require a binder material.

In principle the pressure compaction methods involves the following steps. Firstly, the odour control active materials to be used are combined to form a homogenous mixture. The mixture of actives are than predensified in a predensifier (vertical screw) to remove air, to improve the active densification and to obtain the required particle size. The mixture is then compacted with a compactor (Type: series L 200/5O P smooth rolls) by pressure selected from 10 to 100 KN/cm² to form tablets. The tablets then finally ground to form odour control particles of the required size. As a result of compaction the density of the resultant odour control particles increases by at least 40 %, preferably 100 %, more preferably 150 % and most preferably by at least 200 % of the density of the mixture of odour control actives prior to the compaction process.

### Example 1:

0.74g of zeolite (zealot Wessalith P, available from Degussa) having a particle size of 0.0035mm, 1.0g of silica (silica syloblanc 82, available from Grace) having a particle size of 0.009mm and 0.74g of AGM (AGM -Aquaic L74, available from Shokubay) having a particle size of 0.45mm are mixed together. The mixture is then ground, then pre-compressed and air eliminated therefrom. The mixture is then compacted using a high pressure compactor roll press (available from Hosakawa Bepax GmbH, Germany) to produce a tablet. The tablets are then pre-broken into chips or flakes which are then ground to form the odour control particles of the desired size. The particles are then screened by passing through a sieve and the oversized and fine particles are recycled for reprocessing. The zeolite, silica and AGM mixture has a density of 0.280g/cm³ prior to compaction and a density of 0.550g/cm³ after the compaction process.

### Example 2:

As for example 1, except that in addition to the odour control active materials described above, 0.5g of EDTA (available from BASF, Germany) was added.

### Examples: Absorbent articles

The sanitary napkins used in the following examples were Always (Always is a registered Trade Mark) as sold by the Procter & Gamble Company. Each napkin was opened by cutting the wrap around the perforated coverstock at its bottom face approximately along a longitudinal edge of the release paper which covers the external adhesive layer. The side of the absorbent fibrous core is then exposed by slightly shifting the water impermeable plastic bottom layer and subsequently, the fibrous core is split into two halves, each having approximately the same thickness, along a plane which is parallel to the plane of the napkin itself. The particulate odour control system is homogeneously distributed between these two fibrous layers which are then joined together to reconstitute the absorbent core.

The water impermeable inner backsheet is then put back into its original position and the wrap around perforated coverstock is sealed along the cut by means of a e.g. a double sided adhesive tape.

Samples were produced using the method above, containing the odour control systems as described herein above. A commercially available Always sanitary napkin without modification was used as a reference.

## Claims

1. Odour control particles for absorbent articles, said odour control particles having a particle size and said odour control particles comprising at least one odour control active material wherein the density of said odour control active material is less than the density of said odour control particles, and wherein said odour control active materials are compacted in the absence of a binder to form said odour control particles.

2. Odour control particles according to claim 1, wherein said odour control particles have a density of at least 40% greater than the density of said odour control active material.

3. Odour control particles according to any one of the preceding claims, wherein, said odour control particles have an average particle size of from 0.1 mm to 0.7mm.

4. Odour control particles according to anyone of the preceding claims, wherein, said odour control particles have a density of at least 100 % greater than the density of said odour control active material.

5. Odour control particles according to claim 1, wherein said odour control active materials are selected from zeolites, silica, absorbent gelling material, chelants, carbon and mixtures thereof.

6. Odour control particles according to claim 5, wherein said odour control active materials are zeolite, silica and absorbent gelling material.

7. An absorbent article comprising from 0.2g to 15g, preferably from 0.5g to 5g by weight of said odour control particles, according to any one of the preceding claims.

8. An absorbent article according to claim 7, comprising a liquid pervious topsheet an absorbent core and a liquid impervious backsheet, wherein said core is intermediate said backsheet and said topsheet and wherein said backsheet is moisture vapour permeable.

9. An absorbent article according to claim 7, wherein said article is a sanitary napkin or a panty liner.

10. A process to form said odour control particles according to any one of the claims 1 to 6, comprising the steps of:
i) mixing said odour control active materials
ii) predensifying said materials
iii) dry pressure compacting said odour control active materials to form tablets
iv) grinding said tablets to form said odour control particles.

## Patentansprüche

1. Geruchs- Regulierungsteilchen für absorbierende Artikel, wobei die Geruchs-Regulierungsteilchen eine Teilchengröße haben und die Geruchs- Regulierungsteilchen wenigstens ein aktives Geruchs- Regulierungsmaterial aufweisen, wobei die Dichte des aktiven Geruchs- Regulierungsmaterials geringer ist als die Dichte der Geruchs- Regulierungsteilchen und wobei die aktiven Geruchs- Regulierungsmaterialien bei Abwesenheit eines Binders so verdichtet sind, daß sie die Geruchs- Regulierungsteilchen bilden.

2. Geruchs- Regulierungsteilchen nach Anspruch 1, in welchen die Geruchs- Regulierungsteilchen eine um wenigstens 40 % größere Dichte haben als die Dichte des aktiven Geruchs- Regulierungsmaterials.

3. Geruchs- Regulierungsteilchen nach einem der vorstehenden Ansprüche, in welchen die Geruchs- Regulierungsteilchen eine mittlere Teilchengröße von 0,1 mm bis 0,7 mm haben.

4. Geruchs- Regulierungsteilchen nach einem der vorstehenden Ansprüche, in welchen die Geruchs- Regulierungsteilchen eine um wenigstens 100% größere Dichte haben als die Dichte des aktiven Geruchs- Regulierungsmaterials.

5. Geruchs- Regulierungsteilchen nach Anspruch 1,
in welchen die aktiven Geruchs- Regulierungsmaterialien ausgewählt sind aus Zeolithen, Silika, absorbierendes Geliermaterial, Chelatbildner, Kohlenstoff und Mischungen davon.

6. Geruchs- Regulierungsteilchen nach Anspruch 5,
in welchen die aktiven Geruchs- Regulierungsmaterialien Zeolith, Silika und absorbierendes Geliermaterial sind.

7. Absorbierender Artikel mit von 0,2 g bis 15 g, vorzugsweise 0,5 g bis 5 g Gewichtsanteil der Geruchs- Regulierungsteilchen gemäß einem der vorstehenden Ansprüche.

8. Absorbierender Artikel nach Anspruch 7, mit einer flüssigkeitsdurchlässigen Oberschicht, einem absorbierenden Kern und einer flüssigkeitsundurchlässigen Unterschicht, wobei der Kern zwischen der Unterschicht und der Oberschicht liegt und wobei die Unterschicht wasserdampfdurchlässig ist.

9. Absorbierender Artikel nach Anspruch 7, in welchem der Artikel eine Damenbinde oder eine Höscheneinlage ist.

10. Verfahren, um die Geruchs- Regulierungsteilchen gemäß einem der Ansprüche 1 bis 6 zu bilden, mit den Schritten:
i) Mischen der aktiven Geruchs- Regulierungsmaterialien
ii) Vorverdichten der Materialien
iii) trockenes Druckverdichtung der aktiven Geruchs- Regulierungsmaterialien, um Tabletten zu bilden
iv) Mahlen der Tabletten, um die Geruchs- Regulierungsteilchen zu bilden.

## Revendications

1. Particules de limitation des odeurs pour des articles absorbants, lesdites particules de limitation des odeurs ayant une dimension de particules et lesdites particules de limitation des odeurs comprenant au moins un matériau actif de limitation des odeurs, dans lesquelles la densité dudit matériau actif de limitation des odeurs est inférieure à la densité desdites particules de limitation des odeurs et dans lesquelles lesdits matériaux actifs de limitation des odeurs sont compactés en l'absence d'un liant pour former lesdites particules de limitation des odeurs.

2. Particules de limitation des odeurs selon la revendication 1, dans lesquelles lesdites particules de limitation des odeurs ont une densité d'au moins 40 % supérieure à la densité dudit matériau actif de limitation des odeurs.

3. Particules de limitation des odeurs selon l'une quelconque des revendications précédentes, dans lesquelles lesdites particules de limitation des odeurs ont une dimension de particule moyenne de 0,1 mm à 0,7 mm.

4. Particules de limitation des odeurs selon l'une quelconque des revendications précédentes, dans lesquelles lesdites particules de limitation des odeurs ont une densité d'au moins 100 % supérieure à la densité dudit matériau actif de limitation des odeurs.

5. Particules de limitation des odeurs selon la revendication 1, dans lesquelles lesdits matériaux actifs de limitation des odeurs sont choisis parmi les zéolites, la silice, un matériau gélifiant absorbant, les agents de chélation, du carbone et leurs mélanges.

6. Particules de limitation des odeurs selon la revendication 5, dans lesquelles lesdits matériaux actifs de limitation des odeurs sont la zéolite, la silice et un matériau gélifiant absorbant.

7. Article absorbant comprenant de 0,2 g à 15 g, de préférence de 0,5 g à 5 g en poids desdites particules de limitation des odeurs, selon l'une quelconque des revendications précédentes.

8. Article absorbant selon la revendication 7, comprenant une feuille de dessus perméable aux liquides, une âme absorbante et une feuille de fond imperméable aux liquides, dans lequel ladite âme est située entre ladite feuille de fond et ladite feuille de dessus et dans lequel ladite feuille de fond est perméable à la vapeur humide.

9. Article absorbant selon la revendication 7, dans lequel ledit article est une serviette hygiénique ou une garniture de culotte.

10. Procédé pour former lesdites particules de limitation des odeurs selon l'une quelconque des revendications 1 à 6, comprenant les étapes consistant à :
i) mélanger lesdits matériaux actifs de limitation des odeurs,
ii) prédensifier lesdits matériaux,
iii) compacter sous pression à sec lesdits matériaux actifs de limitation des odeurs pour former des comprimés,
iv) broyer lesdits comprimés pour former lesdites particules de limitation des odeurs.
